(19) Europäisches Patentamt
European Patent Office
Office européen des brevets

(11) Veröffentlichungsnummer: **0 213 326 B1**

(12) # EUROPÄISCHE PATENTSCHRIFT

(45) Veröffentlichungstag der Patentschrift: **06.03.91**

(51) Int. Cl.5: **C07D 207/325**, C07D 417/04, C07D 403/04, A61K 31/40

(21) Anmeldenummer: **86109242.7**

(22) Anmeldetag: **07.07.86**

(54) **2,5-Dimethylpyrrolderivative, ihre Herstellung und ihre Verwendung.**

(30) Priorität: **02.08.85 DE 3527791**

(43) Veröffentlichungstag der Anmeldung:
**11.03.87 Patentblatt 87/11**

(45) Bekanntmachung des Hinweises auf die Patenterteilung:
**06.03.91 Patentblatt 91/10**

(84) Benannte Vertragsstaaten:
**AT BE CH DE FR GB IT LI NL SE**

(56) Entgegenhaltungen:
**DE-A- 1 811 832**
**DE-A- 2 121 831**
**US-A- 2 632 762**
**US-A- 3 702 331**

**CHEMICAL ABSTRACTS, Band 95, Nr. 21, 23. November 1981, Seite 636, Zusammenfassung Nr. 187063z, Columbus, Ohio, US; & JP-A-81 68 666 (MARISHITA PHARMACEUTICAL CO. LTD.) 09-06-1981**

(73) Patentinhaber: **CASSELLA Aktiengesellschaft**
**Hanauer Landstrasse 526**
**W-6000 Frankfurt am Main 61(DE)**

(72) Erfinder: **Zoller, Gerhard, Dr.**
**Höhenstrasse 8**
**W-6369 Schöneck(DE)**
Erfinder: **Beyerle, Rudi, Dr.**
**An der Pfaffenmauer 44**
**W-6000 Frankfurt am Main 60(DE)**
Erfinder: **Schindler, Ursula, Dr.**
**Reinhardswaldweg 1**
**W-6082 Mörfelden-Walldorf(DE)**
Erfinder: **Nitz, Rolf-Eberhard, Dr.**
**Heinrich-Bingemer-Weg 64**
**W-6000 Frankfurt am Main 60(DE)**
Erfinder: **Martorana, Piero, Dr.**
**Kaiser-Friedrich-Promenade 108**
**W-6380 Bad Homburg(DE)**

(74) Vertreter: **Urbach, Hans-Georg, Dr. et al**
**Hanauer Landstrasse 526**
**W-6000 Frankfurt am Main 61(DE)**

EP 0 213 326 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

TETRAHEDRON, Band 26, Nr. 6, März 1970, Seiten 1393-1400, Pergamon Press, GB; G. DESIMONI et al.: "Polynuclear isoxazole types-VI. Reaction of 3-phenyl-4,5-diaminoisoxazole: isoxazolo [4.5-b][1.4]diazepines and isoxazolo[4.5-d]v-triazoles"

JOURNAL OF HETEROCYCLIC CHEMISTRY, Band 10, Nr. 1, Februar 1973, Seiten 181-185; E. ABUSHANAB et al.: "Synthetic studies in the isoxazolo[4,5-b]pyrazine system"

## Beschreibung

Die vorliegende Erfindung betrifft 2,5-Dimethylpyrrolderivate der Formel I

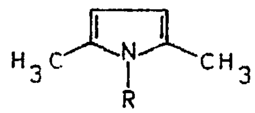

( I )

worin

R Alkyl mit 1 bis 3 C-Atomen, das substituiert ist durch $-NH_2$, Acylamino der Formel

-NH-CX-$R^1$

oder Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, die gegebenenfalls durch eine Carboxylgruppe, Alkoxycarbonyl mit 1 bis 4 c-Atomen in der Alkoxygruppe, Alkyl mit 1 bis 4 C-Atomen oder aliphatische heterocyclische Reste auch durch eine Ketofunktion substituiert sein können; oder Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, die gegebenenfalls durch eine Carboxylgruppe, Alkoxycarbonyl mit 1 bis 4 C-Atomen in der Alkoxygruppe, Alkyl mit 1 bis 4 C-Atomen in der Alkoxygruppe, Alkyl mit 1 bis 4 C-Atomen oder aliphatische heterocyclische Reste auch durch eine Ketofunktion substituiert sein können, bedeutet; X für ein Sauerstoff- oder Schwefelatom steht; $R^1$ Wasserstoff; Alkyl mit 1 bis 5 C-Atomen, das gegebenenfalls substituiert ist durch - $NH_2$, Monoalkylamino mit 1 bis 4 C-Atomen, Dialkylamino mit insgesamt 2 bis 6 C-Atomen, einen N-Pyrrolidinyl-, N-Piperidinyl-, N-Morhpolinyl-, N-Thiomorpholinyl- oder durch einen in 4-Stellung gegebenenfalls durch Alkyl mit 1 bis 4 C-Atomen, Phenyl, Toluyl, Chlorphenyl oder Methoxy- oder EthoxyPhenyl substituierten Piperazin-1-yl-Rest, durch Alkoxy mit 1 bis 4 C-Atomen oder durch Phenoxy, das gegebenenfalls substituiert ist durch eine Aminogruppe, Monoalkylamino mit 1 bis 4 C-Atomen, Dialkylamino mit insgesamt 2 bis 6 C-Atomen, Alkanoylamino mit 1 bis 6 C-Atomen, Alkyl mit 1 bis 4 C-Atomen, Alkoxy mit 1 bis 4 C-Atomen, Halogen, Hydroxy, Nitro, Cyan, Carboxy oder Alkoxycarbonyl mit 1 bis 4 C-Atomen in der Alkoxygruppe;

Cycloalkyl mit 5 bis 7 c-Atomen;

Phenyl, das gegebenenfalls wie der oben bezeichnete Phenoxyrest substituiert sein kann; Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolidin, Oxazol, Oxazolin, Oxazolidin, Thiazol, Thiazolin, Thiazolidin, Pyrimidin, Pyridazin, Pyrazin, Piperazin, Morpholin, Thiomorpholin, Diazepin, Oxazepin, Thiazepin, Perhydrodiazepin, -oxazepin und -thiazepin, die gegebenenfalls durch eine Carboxylruppe, Alkoxycarbonyl mit 1 bis 4 C-Atomen in der Alkoxyruppe, Alkyl mit 1 bis 4 C-Atomen oder aliphatische heterocyclische Reste auch durch eine Ketofunktion substituiert sein können, eine Aminogruppe ($-NH_2$);

oder Phenylamino, dessen Phenylkern gegebenenfalls durch Chlor, Alkyl mit 1 oder 2 C-Atomen, Alkoxy mit 1 oder 2 C-Atomen, Carboxy oder Alkoxycarbonyl mit 1 oder 2 C-Atomen in der Alkoxygruppe substituiert sein kann, bedeutet, sowie deren pharmakologisch akzeptablen Säureadditionssalze. Steht in einer für R stehenden Acylaminogruppe der Formel -NH-CX-$R^1$ das Symbol X für ein Schwefelatom, so bedeutet $R^1$ vorzugsweise eine Aminogruppe ($-NH_2$) oder Phenylamino, dessen Phenylkern gegebenenfalls durch Chlor, Alkyl mit 1 oder 2 C-Atomen, Alkoxy mit 1 oder 2 C-Atomen, Carboxy oder Alkoxycarbonyl mit 1 oder 2 c-Atomen in der Alkoxygruppe substituiert ist.

Ein für $R^1$ stehender Phenylrest und ein als Substituent an einer für $R^1$ stehenden Alkylgruppe gebundener Phenoxyrest ist bevorzugt substituiert durch eine Aminogruppe, Monoalkylamino mit 1 oder 2 C-Atomen, Dialkylamino mit insgesamt 2 bis 4 c-Atomen, Alkanoylamino mit 1 oder 2 C-Atomen, Alkyl mit 1 oder 2 C-Atomen, Alkoxy mit 1 oder 2 C-Atomen, Fluor, Chlor oder Brom, insbesondere Fluor oder Chlor, Hydroxy, Nitro, Cyan, Carboxy oder Alkoxycarbonyl mit 1 oder 2 C-Atomen in der Alkoxygruppe.

Bevorzugte Substituenten für die genannten Phenylkerne sind Chlor, Alkyl und Alkoxy mit 1 oder 2 C-Atomen, insbesondere Methyl oder Methoxy, Carboxy und Alkoxycarbonyl mit 1 oder 2 C-Atomen in der Alkoxygruppe.

In einem an die Piperazingruppe gebundenen Toluyl-, Chlorphenyl- oder Alkoxyphenylrest können die Substituenten, bezogen auf den Piperazinrest, in 2-, 3-oder 4-Stellung, vorzugsweise in 2- oder 4-Stellung, stehen.

Spezielle, besonders bevorzugte Reste R sind Alkylreste mit 1 bis 3 C-Atomen, die durch einen der folgenden Substituenten substituiert sind: Formylamino, Acetylamino, Propionylamino, Isopropionylamino, Butyrylamino,

4-Chlorphenoxyacetylamino, (2-Oxopyrrolidin-1-yl)-acetylamino, N,N-Dimethylamino-acetylamino, L-Thiazolidin-4-yl-carbonylamino, 4-Chlorbenzoylamino, 5-Oxoperhydro-(1,4)-thiazepin-3-yl-carbonylamino, Aminocarbonylamino, 4-Chlorphenylaminocarbonylamino, 1-Acetyl-L-pyrrolidin-2-yl-carbonylamino, 1-Ethyl-(pyrrolidin-2-yl), 2-Oxo-pyrrolidin-1-yl, 2-Oxo-perhydro-azepin-3-yl

Weitere wertvolle Substituenten eines für R stehenden Alkylrestes mit 1 bis 3 C-Atomen sind z.B.: die Aminogruppe, 3,4-Dimethoxybenzoylamino, 2-(4-(2-Methoxyphenyl)-piperazinyl)-acetylamino, Isobutyrylamino, 3-Tert.-butoxycarbonyl-thiazolodin-4-yl-carbonylamio, 4-Ethoxycarbonylphenylamino-carbonylamino, 4-Ethoxycarbonylphenylamino-thiocarbonylamino, 4-Carboxyhenylaminocarbonylamino, 4-Carboxyphenylamino-thiocarbonylamino, Thiazolidin-4-yl, 2-Oxo-pyrrolidin-1-yl, 2-Methylpyrrol-4-yl.

Ein wertvoller für R stehender Heterocyclus ist beispielsweise der 5-Oxo-perhydro-(1,4)-thiazepin-3-yl-Rest.

Besonders bevorzugte erfindungsgemäße Dimethylpyrrolderivate sind solche, die zwei oder mehrere der oben genannten bevorzugten Merkmale aufweisen.

Die Herstellung der erfindungsgemäßen Verbindungen der allgemeinen Formel I

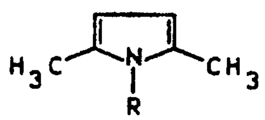

(I),

worin R die oben angegebenen Bedeutungen hat, bzw. ihrer physiologisch verträglichen Säureadditionssalze kann in der Weise erfolgen, daß man Acetonylaceton mit primären Aminen der Formel (II): $H_2N$-R oder ihren Säureadditionssalzen in geeigneten Lösungsmitteln bei Temperaturen von 20 bis 150 °C, vorzugsweise unterhalb 100 °c, insbesondere bei 40 bis 20 °C, umsetzt und, sofern R ein durch einen Rest der Formel

-NH-CX-$R^1$

substituierter Alkylrest mit 1 bis 3 C-Atomen ist, gewünschtenfalls den Acylrest -CX-$R^1$ in an sich bekannter Weise hydrolytisch abspaltet.

Gewünschtenfalls, z.B. bei Einsatz besonders niedrig siedender Lösungsmittel kann die Ringschlußreaktion auch unter Druck oberhalb des Siedepunktes des Reaktionsgemisches ausgeführt werden. Bevorzugt wird jedoch unterhalb oder bei der Siedetemperatur des eingesetzten Lösungsmittels gearbeitet.

Geeignete Lösungsmittel sind z.B. Alkohole, insbesondere solche mit 1 bis 6 C-Atomen, wie z.B. Methanol, Ethanol; i und n-Propanol, i-, sec- und tert-Butanol, n-, i-, sec-, tert-Pentanol, n-Hexanol, Cyclopentanol, Cyclohexanol; Ether, insbesondere solche mit 2 bis 8 C-Atomen im Molekül, wie z.B. Diethylether, Methylethylether, Di-n-propylether, Diisopropylether, Methyl-n-Butylether, Ethylpropylether, Dibutylether, Tetrahydrofuran, 1,4-Dioxan, 1,2-Dimethoxyethan, Bis-ß-methoxyethylether; Polyether, wie z.B. Polyethylenglykole mit einem Molekulargewicht bis ca. 600; Oligoethylen-glykol-dimethylether, wie z.B. Pentaglyme; aliphatische Carbonsäuren, insbe sondere Ameisen- und Essigsäure; Glykole und teilweise veretherte Glykole, wie z.B. Ethylenglykol, Propylenglykol, Trimethylenglykol, Ethylenglykol-monomethylether, Ethylenglykol-monoethylether, Diethylenglykol-monoethylether; aliphatische Kohlenwasserstoffe, wie z.B. niedrigund hochsiedende Petrolether; aromatische Kohlenwasserstoffe, wie z.B. Benzol, Toluol, 0-, m- und p-Xylol; halogenierte aliphatische oder aromatische Kohlenwasserstoffe, wie z.B. Methylenchlorid, Chloroform, Tetrachlorkohlenstoff, Chlorbenzol, Dichlorbenzol; Nitrile, wie z.B. Acetonitril; Amide, wie z.B. Dimethylformamid, N-Methyl-pyrrolidon; Hexamethylphosphorsäuretriamid; Sulfoxide, wie z.B. Dimethylsulfoxid; Wasser. Auch Gemische verschiedener Lösungsmittel können verwendet werden.

Bei der Herstellung der Verbindungen der Formel I werden die Ausgangskomponenten normalerweise in etwa äquimolaren Mengen eingesetzt. Das Amin II kann auch in Form eines Säureadditionssalzes eingesetzt werden. Die Aufarbeitung der Ansätze erfolgt nach üblichen Methoden. Die Umsetzung kann gegebenenfalls auch in Anwesenheit einer Base oder eines Basengemisches durchgeführt werden. Geeignete Basen sind z.B. tertiäre aliphatische Amine, wie z.B. Triethylamin, Tri-n-propylamin und Tri-isopropylamin, ferner Pyridin, sowie Alkali-carbonate, -hydrogencarbonate.

Die evtl. gewünschte Abspaltung des Acylrestes aus erfindungsgemäßen Verbindungen der Formel I, in denen R ein durch die Gruppe

-NH-CX-$R^1$

substituierter Alkylrest ist, führt zu den entsprechenden Verbindungen, in denen der für R stehende Alkylrest durch eine primäre Aminogruppe substituiert ist. Die Abspaltung wird in an sich bekannter Weise

hydrolytisch durchgeführt. Hierzu werden die Verbindungen mit Wasser oder einem wasserhaltigen organischen Medium in Gegenwart von molaren Mengen einer Base behandelt. Die Behandlungs dauer hängt von der gewählten Temperatur ab. Man kann bei Zimmertemperatur oder, um die Hydrolyse zu beschleunigen, bei erhöhter Temperatur, zweckmäßigerweise bis zur Rückflußtemperatur des flüssigen Hydrolysemediums, arbeiten.

Die Art der zuzusetzenden Base ist im Prinzip belanglos. Diese Reagentien sollen nur eine ausreichend hohe $OH^-$-Konzentration herbeiführen und eine einfache Aufarbeitung der Ansätze gestatten. Die Wahl dieser Mittel kann daher in bekannter Weise erfolgen.

Andererseits können auch zur Herstellung der erfindungsgemäßen Verbindungen der Formel I, in denen R ein durch eine Acylaminogruppe der Formel -NH-C0-$R^1$ substituierter Alkylrest mit 1 bis 3 C-Atomen ist, Aminoalkylpyrrole der Formel III

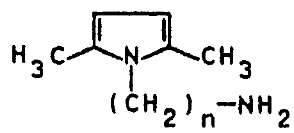

(III),

worin n eine Zahl von 1 bis 3 ist, mit reaktiven Carbonsäurederivaten, abgeleitet von Carbonsäuren der Formel $R^1$-COOH, worin $R^1$ die oben angegebene Bedeutung hat, mit Alkalicyanat bzw. -thiocyanat oder mit Isocyanaten bzw. Isothiocyanaten der Formel

$R^3$-NCX,

worin X Sauerstoff oder Schwefel ist und $R^3$ gegebenenfalls substituiertes Phenyl bedeutet, vorzugsweise Phenyl, das gegebenenfalls durch Chlor, Alkyl mit 1 oder 2 C-Atomen, Alkoxy mit 1 oder 2 C-Atomen, Carboxy oder Alkoxycarbonyl mit 1 oder 2 C-Atomen in der Alkoxygruppe, insbesondere in der oben präzisierten Weise, substituiert ist, acyliert werden.

Als reaktive Carbonsäurederivate eignen sich Carbonsäureester, Carbonsäureanhydride, Carbonsäurechloride oder Carbonsäuren, die in situ aktiviert werden, wie z.B. mit Dicyclohexylcarbodiimid (Houben Weyl 8 ` 522), Oxalylchlorid (GB 2139-225), N,N-Carbonyldiimidazol (J.Med.chem. 1982 ; 620, Synthesis 1982 , 833; Chem.Pharm. Bull. 32 , 5044 (1984)); N,N'-Carbonyldiazolen (Bull. Chem. Soc. Jap. 57 , 3597 (1984)); Kohlensäure-di-(2-pyridyl)-ester (Tetrahedron Lett. 25 , 4943 (1983)); Chlorameisensäureester (Tetrahedron Lett. 24 , 3365 (1983)); Diphosphortetrajodid (Chem. Lett. 1983 , 449); Dialkyldisulfit (Indian J. Chem. 21 . 259 (1982)); Methylethylphosphinsäureanhydrid oder mit anderen reaktiven Agentien.

Bei Einsatz von Isocyanaten bzw. Isothiocyanaten der Formeln $R^3$-NCO bzw. $R^3$-NCS als Acylierungsmittel werden diejenigen erfindungsgemäßen 2,5-Dimethylpyrrolderivate erhalten, in denen R ein durch die Gruppen -HN-CO-NH-$R^3$ bzw. -NH-CS-NH-$R^3$ substituierter Alkylrest ist. Umsetzung der Amine der Formel III mit Alkalicyanaten bzw. -thiocyanaten liefert erfindungsgemäße Verbindungen, in denen R ein durch die Gruppen -NH-C0-$NH_2$ bzw. -NH-CS-$NH_2$ substituierter Alkylrest ist.

Die Umsetzungen werden zweckmäßigerweise in flüssiger Phase durchgeführt, wobei die Anwesenheit eines inerten Lösungsmittels vorteilhaft ist.

Werden enantiomerenreine Carbonsäurederivate oder Amine eingesetzt, so können auch die erfindunysgemäßen Verbindungen der Formel I als enantiomerenreine Verbindungen erhalten werden.

Sofern die 2,5-Dimethyl-pyrrol Derivate der Formel I basische Reste enthalten, bilden sie mit anorganischen oder organischen Säuren Säureadditionssalze. Zur Bildung derartiger Säureadditionssalze sind anorganische und organische Säuren geeignet. Geeignete Säuren sind bei spielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Nicotin-, Isonicotin-, Methansulfon-, p-Toluolsulfon-, Zitronen-oder Adipin-Säure. Pharmakologisch annehmbare Säureadditionssalze werden bevorzugt. Die Säureadditionssalze werden wie üblich durch Vereinigung der Komponenten, zweckmäßigerweise in einem geeigneten Lösungsmittel oder Verdünnungsmittel, hergestellt. Bei der Synthese der Verbindungen der Formel I können zunächst die Säureadditionssalze im Zuge der Aufarbeitung anfallen. Aus den Säureadditionssalzen können die freien Verbindungen der allgemeinen Formel I gewünschtenfalls in bekannter Weise, z.B. durch Auflösen oder Suspendieren in Wasser und Alkalischstellen, z.B. mit Natronlauge, und anschließendes Isolieren, gewonnen werden.

Die erfindungsgemäßen Verbindungen der Formel I und ihre pharmakologisch annehmbaren Säureadditionssalze besitzen wertvolle pharmakologische Eigenschaften. Sie sind zentralwirksam, beispielsweise zeigen sie encephalotrope und nootrope Wirkungen und dienen zur Behandlung von Krankheiten der

Gehirnfunktionen wie cerebraler Insuffizienz, cerebraler Alterungsvorgänge, verminderter Gedächtnisleistung wie sie auch bei der Alzheimer Krankheit oder der Multi-Infarkt Demenz oder bei verminderter Lernleistung auftreten. Sie sind den bisher bekannten Verbindungen gleicher Wirkungsrichtung überraschenderweise erheblich überlegen. Sie zeigen eine ausgezeichnete Wirksamkeit in verschiedenenartigen Tests, wie z.B. bei der Verlängerung der Uberlebenszeit unter Natriumnitrit-Hypoxie nach Gibsen und Blass (J. Neurochemistry 27 , 37 (1976)), bei der Verbesserung der stickstoffinduzierten Hypoxietoleranz, wobei Versuchstiere nach Prämedikation mit den untersuchten Präparaten mit reinem Stickstoff beatmet werden und die Verlängerung des Zeitraumes zwischen Einsatz der Beatmung und elektrischer Neutralität des Elektroencephalogramms sowie die Letalität gemessen werden.

Auch in Tests, die direkt auf die Erfassung der Lern-und Gedächtnisleistung abzielen, wie z.B. den bekannten "avoidance"-Tests, sind die erfindungsgemäßen Produkte sehr gut wirksam.

Die Prüfung in den genannten und einer Reihe weiterer Tests, wie z.B. dem $\gamma$-Butyrolacton-Test, ergibt, daß die erfindungsgemäßen Verbindungen in niedrigen Dosen bei geringer Toxizität überraschenderweise ein besonders günstiges, bei bekannten Präparaten in dieser Form nicht vorliegendes Wirkprofil aufweisen.

Die Verbindungen der Formel I und ihre physiologisch verträglichen Salze stellen somit eine Bereicherung der Pharmazie dar.

Die Verbindungen der Formel I und die vorgenannten Verbindungen und ihre pharmakologisch annehmbaren Säureadditionssalze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder einer vorgenannten Verbindung oder eines Säureadditionssalzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittel können oral, z.B. in Form von Pillen, Tabletten, Lacktabletten, Dragees, Granulaten, Hart und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glycerin, Polyole, pflanzliche Öle etc.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks-oder Aromatisierungs-, Dickungs-, Verdünnungs--Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Di hydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lanato-Glykoside; Coronardilatatoren, wie Carbocromen, Dipyridamol, Nifedipin und Perhexilin, antianginöse Verbindungen, wie Isosorbiddinitrat, Isosorbidmononitrat, Glycerolnitrat, Molsidomin und Verapamil, ß-Blocker, wie Propranolol, Oxprenolol, Atenolol, Metoprolol und Penbutolol. Darüberhinaus lassen sich die Verbindungen mit anderen nootrop wirksamen Substanzen, wie z.B. Piracetam, oder ZNSaktiven Substanzen, wie Pirlindol, Sulpirid etc. kombinieren.

Die Dosierung kann innerhalb weiter Grenzen variieren und ist in jedem einzelnen Fall den individuellen Gegebenenheiten anzupassen. Im allgemeinen ist bei der oralen Verabreichung eine Tagesdosis von etwa 0, 1 bis 1 mg/kg, vorzugsweise 0,3 bis 0,5 mg/kg Körpergewicht zur Erzielung wirksamer Ergebnisse angemessen, bei intravenöser Application beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3 oder 4, Teilverabreichungen aufgeteilt. Gegebenenfalls kann es, je nach individuellem Verhalten erforderlich werden, von der angegebenen Tagesdosis nach oben

oder nach unten abzuweichen. Pharmazeutische Präparate enthalten normalerweise 0,1 bis 50 mg, vorzugsweise 0.5 bis 10 mg Wirkstoff der Formel I oder der genannten Verbindungen oder eines pharmakologisch annehmbaren Salzes pro Dosis.

Beispiel 1:

2,5-Dimethyl-1-(2-(2-Oxo-pyrrolidin-1-yl)-ethyl)-pyrrol

5,7g (0.05 mol) Acetonylaceton und 6,4 g (0,05 mol) 1-(2-Aminoethyl)-pyrrolidin-2-on werden in 70 ml Methanol

2 h unter Rückfluß erhitzt. Das Reaktionsgemisch wird dann eingeengt, und nach dem Einengen wird anschließend das rohe Reaktionsprodukt durch Zusatz von Petrolether ausgefällt. Das Rohprodukt wird aus Diethylether umkristallisiert.

| | |
|---|---|
| Ausbeute: | 3,2 g (31 % der Theorie), |
| Schmelzpunkt: | 66-68 °C |
| Elementaranalyse: | $C_{12}H_{18}N_2O$ (206,29) |
| berechnet: | C 69,9 H 8,8 N 13,6 O 7,8 |
| gefunden: | C 69,4 H 8,7 H 13,7 O 8,1 |

Beispiel 2:

2.5-Dimethyl-1-(2-Oxo-perhydro-azepin-3-yl)-pyrrol

21g (0,1 mol) einer 60%igen wäßrigen 3-Amino-ε-Caprolactam-Lösung werden mit 200 ml Chloroform azeotrop entwässert, mit 11,4 g (0,1 mol) Acetonylaceton in 150 ml Ethylenglykolmonomethylether versetzt und 2 h bei 80 °C gerührt. Nach dem Einengen des Reaktionsgemisches wird mit Ether verrührt und der erhaltene Niederschlag abgesaugt.

| | |
|---|---|
| Ausbeute: | 16,4 g (79 % der Theorie), |
| Schmelzpunkt: | 150-152 °C |
| Elementaranalyse: | $C_{12}H_{18}N_2O$ (206,29) |
| berechnet: | C 69,9 H 8,8 N 13,6 O 7,8 |
| gefunden: | C 69,3 H 8,4 H 13,5 O 8,3 |

Beispiel 3:

2,5-Dimethyl-1-((l-Ethylpyrrolidin-2-yl)-methyl)-pyrrol

11,4 g (0, 1 mol) Acetonylaceton und 12,8 g (0,1 mol) 1-Ethyl-2-aminomethylpyrrolidin werden in 120 ml Isopropanol 1 h unter Rückfluß erhitzt. Man engt das Reaktionsgemisch ein und destilliert es fraktioniert im Vakuum.

| | |
|---|---|
| Ausbeute: | 13,2 g (64 % der Theorie), |
| Siedepunkt: | 73 °C / 0,4 mbar |
| Elementaranalyse: | $C_{13}H_{22}N_2$ (206,34) |
| berechnet: | C 75,7 H 10,7 H 13,6 |
| gefunden: | C 75,9 H 10,3 H 13,1 |

Beispiel 4:

2,5-Dimethyl-1-((5-methyl-pyrrol-2-yl)-methyl)-pyrrol

11,4 g (0,1 mol) Acetonylaceton und 11 g (0,1 mol) 5-Methyl-2-aminomethyl-pyrrol werden in 100 ml 1,2-Dimethoxyethan 1 h unter Rückfluß erhitzt. Man engt das Reaktionsgemisch ein und destilliert es fraktioniert im Vakuum.

| | |
|---|---|
| Ausbeute: | 5,8 g (31 % der Theorie), |
| Siedepunkt: | 90 °C / 0,133 mbar |
| Elementaranalyse: | $C_{12}H_{16}N_2$ (188,28) |
| berechnet: | C 76,6 H 8,7 H 14,9 |
| gefunden: | C 75,9 H 9,0 H 14,3 |

Beispiel 5:

2,5-Dimethyl-1-(2-acetylaminoethyl)-Pyrrol

171,2 g (1,5 mol) Acetonylaceton und 153,2 g (1,5 mol) 1-Acetyl-ethylendiamin werden in 1,8 1 Ethanol 3 h unter Rückfluß erhitzt. Man engt das Reaktionsgemisch ein und kristallisiert den Rückstand aus Toluol um.

| | |
|---|---|
| Ausbeute: | 259,3 g (96 % der Theorie), |
| Schmelzpunkt: | 106-107 °C |
| Elementaranalyse: | $C_{10}H_{16}N_2O$ (180,25) |
| berechnet: | C 66,6 H 8,9 N 15,5 O 8,9 |
| gefunden: | C 66,8 H 8,8 N 15,6 O 8,9 |

Beispiel 6:

1-(2-Aminoethyl)-2,5-dimethyl-pyrrol

251, 1 g (1,39 mol) 2,5-Dimethyl-1-(2-acetylaminoethyl)-pyrrol, hergestellt gemäß Beispiel 5, und 390,8 g (6,97 mol) Kaliumhydroxid werden in 2,8 1 Ethanol und 280 ml Wasser 4 h in einer Stickstoffatmosphäre unter Rückfluß

erhitzt. Nach dem Einengen des Ansatzes wird die wäßrige Phase mit Methylenchlorid extrahiert und der Extrakt fraktioniert destilliert.

| | |
|---|---|
| Ausbeute: | 150,2 g (78 % der Theorie), |
| Siedepunkt: | 70 °C / 0,267 mbar |

Beispiel 7:

N-(2-(2,5-Dimethylpyrrol-1-yl)-ethyl)-N'-(4-ethoxycarbo nylphenyl)-harnstoff

5,5 g (0,04 mol) 1-(2-Aminoethyl)-2,5-dimethylpyrrol werden in 20 ml Methylenchlorid gelöst. Zu dieser Lösung werden 7,6 g (0,04 mol) 4-Ethoxycarbonylphenylisocyanat in 20 ml Methylenchlorid bei Raumtemperatur zugetropft. Der ausfallende Niederschlag wird abgesaugt und aus Ethanol umkristallisiert.

| | |
|---|---|
| Ausbeute: | 7,3 g (55 % der Theorie), |
| Schmelzpunkt: | 181-182 °C |
| Elementaranalyse: | $C_{18}H_{23}N_3O_3$ (329,40) |
| berechnet: | C 65,6 H 7,0 N 12,8 O 14,6 |
| gefunden: | C 65,0 H 7,2 N 12,9 O 14,8 |

Beispiel 8:

N-(2-(2,5-Dimethylpyrrol-1-yl)-ethyl)-N'-(4-carboxy phenyl)-harnstoff

3,3 9 (0,01 mol) N-(2-(2,5-Dimethylpyrrol-1-yl)-ethyl)-N'-(4-ethoxycarbonylphenyl)-harnstoff, hergestellt nach Beispiel 7, werden mit 12 ml Methanol und 10 ml 1n Natronlauge 10 h bei Raumtemperatur gerührt und mit verdünnter Salzsäure angesäuert. Das ausgefallene Produkt wird abgesaugt und getrocknet.

| | |
|---|---|
| Ausbeute: | 1,5 g (50 % der Theorie), |
| Schmelzpunkt: | 248-250 °C |
| Elementaranalyse: | $C_{16}H_{19}N_3O_3$ (301,35) |
| berechnet: | C 63,8 H 6,4 N 13,9 O 15,9 |

gefunden: C 63,4 H 6,2 N 13,9 O 16,3

Beispiel 9:

N-(2-(2,5-Dimethylpyrrol-1-yl)-ethyl)-N'-(4-ethoxycarbo nylphenyl)-thioharnstoff

8,3 g (0,04 mal) 4-Ethoxycarbonylphenyl-isothiocyanat in 20 ml Ethylenglykoldimethylether werden bei Raumtemperatur zu 5,5 g (0,04 mol) 1 (2-Aminoethyl)-2,5-dimethylpyrrol in 20 ml Ethylenglykoldimethylether zugetropft. Das ausgefallene Produkt wird abgesaugt und aus Methanol umkristallisiert.

| | |
|---|---|
| Ausbeute: | 5,1 g (37 % der Theorie), |
| Schmelzpunkt: | 155-156 ° C |
| Elementaranalyse: | $C_{18}H_{23}N_3O_2S$ (345,47) |
| berechnet: | C 62,6 H 6,7 N 12,2 O 9,3 S 9,3· |
| gefunden: | C 62,0 H 6,7 N 11,6 O 10,0 S 9,2 |

Beispiel 10:

N-(2-(2,5-Dimethylpyrrol-1-yl)-ethyl)-N'-(4-carboxy phenyl)-thioharnstoff

2,8 g (0,008 mol) N-(2-(2,5-Dimethylpyrrol-1-yl)-ethyl)-N'-(4-ethoxycarbonylphenyl)-thioharnstoff, hergestellt nach Beispiel 9, werden mit 10 ml 1n Natronlauge 5 h bei Raumtemperatur und 15 min unter Rückfluß gerührt. Nach dem Abkühlen wird filtriert, angesäuert und abgesaugt.

| | |
|---|---|
| Ausbeute: | 1,9 g (75 % der Theorie), |
| Schmelzpunkt: | 174-175 ° C |
| Elementaranalyse: | $C_{16}H_{19}N_3O_2S$ (317,41) |
| berechnet: | C 60,5 H 6,0 N 13,2 O 10,1 S 10,1 |
| gefunden | : C 59,9 H 5,9 N 13,6 O 10,7 S 9,9 |

Beispiel 11:

N-(2-(2,5-Dimethylpyrrol-1-yl)-ethyl)-N'-(4-chlorphenyl)-harnstoff

7,7 9 (0,05 mol) 4-Chlorphenylisocyanat, gelöst in 10 ml

Tetrahydrofuran, werden zu 6,9 g (0,05 mol) 1-(2-Aminoethyl)-2,5-dimethylpyrrol, gelöst in 10 ml Tetrahydrofuran, im Verlauf von 30 Minuten bei 20 ° C zugetropft. Man läßt noch 60 Minuten bei 20 ° C nachrühren. Nach Zugabe von Diethylether zum Reaktionsgemisch kristallisiert das, Produkt aus, wird abgesaugt und aus Isopropanol umkristallisiert.

| | |
|---|---|
| Ausbeute: | 8,4 9 (58 % der Theorie), |
| Schmelzpunkt: | 175-177 ° C |
| Elementaranalyse: | $c_{15}H_{18}ClN_3O$ (291,83) |
| berechnet: | C 61,7 H 6,2 Cl 12,2 N 14,4 O 5,5 |
| gefunden: | C 61,0 H 6,4 Cl 12,5 N 14,6 O 5,8 |

Beispiel 12:

2-(2,5-Dimethylpyrrol-1-yl)-ethyl-harnstoff

6,9 g (0,05 mol) 1-(2-Aminoethyl)-2,5-dimethylpyrrol werden in 8 ml Wasser gelöst. Unter Kühlen werden langsam 5 ml konzentrierte Salzsäure und anschließend 4,9 g (0,05 mol) Kaliumcyanat, gelöst in 30 ml Wasser, zugetropft. Der ausgefallene Feststoff wird abgesaugt und aus Toluol/Essigsäureethylester umkristallisiert.

| | |
|---|---|
| Ausbeute: | 3,8 9 (42 % der Theorie), |
| Schmelzpunkt: | 139-141 ° C |
| Elementaranalyse: | $C_9H_{15}N_3O$ (181,24) |

| | |
|---|---|
| berechnet: | C 59,6 H 8,3 N 23,2 O 8,8 |
| gefunden: | C 59,2 H 8,2 H 22,8 o 9, 1 |

Beispiel 13:

4-Chlorphenoxyessigsäure-2-(2,5-dimethyl-pyrrol-1-yl)-ethyl-amid

9,3 9 (0,05 mol) 4-Chlorphenoxyessigsäure, 6,9 g (0,05 mol) 1-(2 Aminoethyl)-2,5-dimethyl-pyrrol und 35 ml Triethylamin werden in 50 ml Dimethylformamid gelöst. Bei -5 bis 0° C werden 20 ml Methylethylphosphinsäureanhydrid zugetropft und 3 h bei Raumtemperatur nachgerührt. Das

Reaktionsgemisch wird auf eiskalte wäßrige Natriumhydrogencarbonatlösung gegossen, mit Methylenchlorid extrahiert und der Extrakt eingeengt. Der Rückstand wird über eine 20 cm Kieselgelsäule mit Methylenchlorid/Essigsäureethylester-Gemisch 1:1 als Laufmittel chromatographiert und mit Ligroin kristallisiert.

| | |
|---|---|
| Ausbeute: | 10,5 g (68 % der Theorie), |
| Schmelzpunkt: | 75-77° C |
| Elementaranalyse: | $C_{16}H_{19}ClN_2O_2$ (306,80) |
| berechnet: | C 62,6 H 6,2 Cl 11,6 N 9,1 O 10,4 |
| gefunden: | C 62,7 H 6,4 Cl 11,7 N 9,2 O 10,4 |

Beispiel 14:

(2-Oxo-pyrrolidin-1-yl)-essigsäure-2-(2,5-dimethylpyrrol-1-yl)-ethyl-amid

14,3 g (0,1 mol) (2-Oxo-pyrrolidin-1-yl)-essigsäure und 16,2 g (0,1 mol) N,N'-Carbonyldiimidazol werden in 70 ml Tetrahydrofuran 15 min unter Rückfluß erhitzt. Nach dem Abkühlen werden 13,8 g (0,1 mol) 1-(2-Aminoethyl)-2,5-dimethylpyrrol in 70 ml Tetrahydrofuran zugetropft. Nach 6 h Rühren bei Zimmertemperatur wird das Reaktionsgemisch eingeengt, mit verdünnter Essigsäure angesäuert und mit Methylenchlorid extrahiert. Der Methylenchlorid-Extrakt wird eingeengt, der Rückstand mit Ether verrührt, abgesaugt und aus Dibutylether umkristallisiert.

| | |
|---|---|
| Ausbeute | : 9,0 g (34 % der Theorie), |
| Schmelzpunkt: | 109-111° C |
| Elementaranalyse: | $C_{14}H_{21}N_3O_2$ (263,34) |
| berechnet: | C 63,9 H 8,0 N 16,0 O 12,2 |
| gefunden: | C 63,8 H 8,0 N 15,9 O 12,2 |

Beispiel 15:

1-Acetyl-tetrahydro pyrrol-2-carbonsäure-2-(2,5-di methylpyrrol-1-yl)-ethylamid

6,3 g (0,04 mol) 1-Acetyl-L Prolin und 6,5 g (0,04 mol)

Carbonyldiimidazol werden in 20 ml DMF 10 min bei 60° C gerührt. Nach Zugabe von 5,5 g (0,04 mol) 1-(2-Aminoethyl)-2,5-dimethylpyrrol wird der Ansatz 3 h bei Raumtemperatur gerührt, dann eingeengt, wie im Beispiel 14 aufgearbeitet und aus Toluol umkristallisiert.

| | |
|---|---|
| Ausbeute: | 5,7 g (51 % der Theorie), |
| Schmelzpunkt: | 131-133° C |
| Elementaranalyse: | $C_{15}H_{23}N_3O_2$ (277,37) |
| berechnet: | C 65,0 H 8,3 N 15,2 O 11,5 |
| gefunden: | C 65,2 H 7,9 H 15,3 O 11,4 |

Beispiel 16:

3,4-Dimethoxybenzoesäure-2-(2,5-dimethyl-pyrrol-1-yl)-ethylamid

9,1 g (0,05 mol) 3`4-Dimethoxybenzoesäure und 8,2 g (0,05 mol) Carbonyldiimidazol werden in 50 ml

Dioxan 15 min bei 60° C gerührt. Nach Zugabe von 6,2 g (0,045 mol) 1-(2-Aminoethyl)-2,5-dimethylpyrrol in 20 ml Dioxan wird der Ansatz 20 h bei Raumtemperatur gerührt, eingeengt, mit wäßriger Natriumhydrogencarbonatlösung versetzt und mit Methylenchlorid extrahiert. Der Extrakt wird über eine Aluminiumoxidsäule chromatographiert und mit Essigsäureethylester/Ether/Ligroin kristallisiert.

| | |
|---|---|
| Ausbeute: | 6,9 g (46 % der Theorie), |
| Schmelzpunkt: | 82-84° C |
| Elementaranalyse: | $C_{17}H_{22}N_2O_3$ (302,37) |
| berechnet: | C 67,5 H 7,3 N 9,3 O 15,9 |
| gefunden: | C 67,3 H 7,2 N 8,8 O 16,6 |

Beispiel 17:

5-Oxo-perhydro-(1,4)-thiazepin-3-carbonsäure-2-(2,5-di methylpyrrol-1-yl)-ethylamid

3,5 9 (0,02 mol) 5-Oxo-perhydro-(1,4)-thiazepin-3-carbonsäure und 2,8g (0,02 mol) 1-(2-Aminoethyl)-2,5-dimethylpyrrol werden in 10 ml Dimethylformamid gelöst. Nach

Zugabe von 4,2 g (0,02 mol) Dicyclohexylcarbodiimid, gelöst in 10ml Methylenchlorid, bei 0° C wird der Ansatz 24 h bei Raumtemperatur gerührt, mit Wasser versetzt und abgesaugt. Das Filtrat wird mit Methylenchlorid extrahiert und der Extrakt über eine Kieselgelsäule mit Essigsäureethylester als Laufmittel chromatographiert.

| | |
|---|---|
| Ausbeute: | 1,2 g (20 % der Theorie) |
| Schmelzpunkt: | 81-183° C |
| Elementaranalyse: | $C_{14}H_{21}N_3O_2S$ (295,41) |
| berechnet: | C 56,9 H 7,2 N 14,2 O 10,8 S 10,9 |
| gefunden: | C 57,4 H 6,9 N 13,8 O 11,2 S 10,8 |

Beispiel 18:

n-Buttersäure-2-(2,5-dimethylpyrrol-1-yl)-ethylamid

zu 5,5 g (0,04 mol) 1-(2-Amino-ethyl)-2,5-dimethylpyrrol, gelost in 20 ml Toluol, werden 6,4 g (0,04 mol) Buttersäureanhydrid, gelöst in 20 ml Toluol, bei Raumtemperatur zugetropft. Man rührt 5 h weiter, engt den Ansatz ein, versetzt ihn mit wäßriger Natriumhydrogencarbonatlösung und extrahiert ihn mit Methylenchlorid. Den Extrakt engt man ein und kristallisiert den erhaltenen Rückstand aus Dibutylether um.

| | |
|---|---|
| Ausbeute: | 4,8g (58 % der Theorie), |
| Schmelzpunkt: | 66-68° C |
| Elementaranalyse: | $C_{12}H_{20}N_2O$ (208,31) |
| berechnet: | C 69,2 H 9,7 N 13,4 O 7,7 |
| gefunden: | C 68,7 H 9,3 N 13,5 O 8,6 |

Beispiel 19:

4-Chlorbenzoesäure-2-(2,5-dimethyl-pyrrol-1-yl-ethylamid

Zu 5,5 g (0,04 mol) 1-(2-Aminoethyl) -2,5-dimethylpyrrol, gelöst in 30 ml Pyridin, werden bei 0° c 7,0 g (0,04 mol) 4 Chlorbenzoylchlorid, gelöst in 20 ml Methylenchlorid, zugetropft. Man rührt ä h bei 0° C, 4 h bei Raumtemperatur, hydrolysiert den Säurechlorid Uberschuß durch Zusatz

von Wasser, extrahiert das Reaktionsprodukt mit Methylenchlorid und engt den Extrakt ein. Das erhaltene Rohprodukt kann aus Ligroin umkristallisiert werden.

| | |
|---|---|
| Ausbeute: | 5,4 g (49 % der Theorie) |
| Schmelzpunkt: | 104-105° C |
| Elementaranalyse: | $C_{15}H_{17}ClN_2O$ (276,82) |
| berechnet: | C 65,1 H 6,2 Cl 12,8 N 10,1 O 5,8 |
| gefunden: | C 65,4 H 6,1 Cl 12,5 N 10,1 O 5,9 |

Beispiel 20:

N-N-Dimethylaminoessigsäure-2-(2,5-dimethylpyrrol-1-yl)-ethylamid

5,2 g (0,05 mol) N,H-Dimethylaminoessigsäure und 8,1 g (0,05 mol) Carbonyldiimidazol werden in 20 ml Ethylenglykoldimethylether 15 min bei 70 °C gerührt. Nach Zugabe von 5,5 g (0,04 mol) 1-(2-Aminoethyl)-2,5-dimethylpyrrol, gelöst in 20 ml Ethylenglykoldimethylether, wird der Ansatz bei Raumtemperatur 5 h weitergerührt, eingeengt, mit Wasser versetzt und zuerst sauer und dann alkalisch extrahiert. Das im Extrakt des alkalisch gestellten Ansatzes enthaltene Produkt wird mit Ligroin kristallisiert.

| | |
|---|---|
| Ausbeute: | 4,3 g (39 % der Theorie), |
| Schmelzpunkt: | 63-65 °C |
| Elementaranalyse: | $C_{12}H_{21}N_3O$ (223,32) |
| berechnet: | C 64,5 H 9,5 N 18,8 O 7,2 |
| gefunden: | C 64,0 H 9,4 N 18,5 O 8,0 |

Beispiel 21:

N-Boc-L-Thiazolidin-4-carbonsäure-2-(2,5-dimethylpyrrol-1-yl)-ethylamid

11,6 g (0,05 mol) N-Boc-L-Thiazolidin-4-carbonsäure und 8,1 g (0,05 mol) Carbonyldiimidazol werden in 10 ml wasserfreiem DMF 10 Minuten bei 60 °C gerührt. Nach Zugabe von 6,9 g (0,05 mol) 1 (2-Aminoethyl)-2,5-dimethylpyrrol,

gelöst in 10 ml DMF, wird der Ansatz 3 h weitergerührt, mit Wasser versetzt und mit Methylenchlorid extrahiert. Der Extrakt wird über eine Kieselgelsäule chromatographiert (Laufmittel: Methylenchlorid/Essigsäureethylester 1:1) und mit Dibutylether kristallisiert.

| | |
|---|---|
| Ausbeute: | 12,0 g (68 % der Theorie), |
| Schmelzpunkt: | 119-121 °C |
| Elementaranalyse: | $C_{17}H_{27}N_3O_3S$ (353,49) |
| berechnet: | C 57,8 H 7,7 N 11,9 O 13,6 S 9, 1 |
| gefunden: | C 58,2 H 7,3 N 12,3 O 13,7 S 9,2 |

(Boc = tert.-Butoxycarbonyl)

Analog den angegebenen Beispielen lassen sich beispielsweise auch die folgenden erfindungsgemäßen Verbindungen herstellen:

a) L-Thiazolidin-4-carbonsäure-2-(2,5-dimethylpyrrol-1-yl)-ethylamid
b) 2,5-Dimethyl-1-(2-formylaminoethyl)-pyrrol
c) 2,5-Dimethyl-1-(2-propionylaminoethyl)-pyrrol
d) 2,5-Dimethyl-1-(3-acetylaminopropyl)-pyrrol Fp: 66-67 °C;
e) 2,5-Dimethyl-1-(3-formylaminopropyl)-pyrrol
f) 2,5-Dimethyl-1-(3-propionylaminopropyl)-pyrrol

In den nachfolgenden Beispielen werden pharmazeutische Präparate beschrieben:

Beispiel A

Emulsionen mit 3 mg Wirkstoff per 5ml können nach folgender Rezeptur hergestellt werden:

| | |
|---|---|
| Wirkstoff | 0,06g |
| Neutralöl | q.s. |
| Natriumcarboxymethylzellulose | 0,6g |
| Polyoxyethylenstearat | q.s. |

| | |
|---|---|
| Reinglyzerin | 0,2 bis 2g |
| Aromastoffe | q.q.s. |
| Wasser (entmineralisiert oder destilliert) | ad 100ml |

Beispiel B

Dragees, enthaltend einen erfindungsgemäßen Wirkstoff und einen anderen therapeutisch wirksamen Stoff:

| | |
|---|---|
| Wirkstoff | 6mg |
| Propranolol | 40mg |
| Milchzucker | 90mg |
| Maisstärke | 90mg |
| sec Calciumphosphat | 34mg |
| lösliche Stärke | 3mg |
| Magnesiumstearat | 3mg |
| kolloidale Kieselsäure | 4mg |
| | 270mg |

Beispiel C

Dragees, enthaltend einen erfindungsgemäßen Wirkstoff und einen anderen therapeutisch wirksamen Stoff:

| | |
|---|---|
| Wirkstoff | 5mg |
| Molsidomin | 5mg |
| Milchzucker | 60mg |
| Maisstärke | 90mg |
| sec Calciumphosphat | 30mg |
| lösliche Stärke | 3mg |
| Magnesiumstearat | 3mg |
| kolloidale Kieselsäure | 4mg |
| | 200mg |

Beispiel D

Kapseln, enthaltend einen erfindungsgemäßen Wirkstoff und einen anderen therapeutisch wirksamen Stoff:

| | |
|---|---|
| Wirkstoff | 5mg |
| Prazosin | 5mg |
| Maisstärke | 185mg |
| | 195mg |

**Beispiel E**

Tabletten können nach folgender Formulierung hergestellt werden:

| | |
|---|---|
| Wirkstoff | 2mg |
| Lactose | 60mg |
| Maisstärke | 30mg |
| lösliche Stärke | 4mg |
| Magnesiumstearat | 4mg |
| | 100mg |

**Beispiel F**

Für die Herstellung von Weichgelatinekapseln mit 5mg Wirkstoff pro Kapsel eignet sich die folgende Zusammensetzung:

| | |
|---|---|
| Wirkstoff | 5mg |
| Mischung von Triglyzeriden aus Kokosöl | 150mg |
| Kapselinhalt | 155mg |

**Beispiel G**

Für die Herstellung von Dragees eignet sich folgende Formulierung

| | |
|---|---|
| Wirkstoff | 1mg |
| Maisstärke | 100mg |
| Lactose | 60mg |
| sec Calciumphosphat | 30mg |
| lösliche Stärke | 3mg |
| Magnesiumstearat | 2mg |
| kolloidale Kieselsäure | 4mg |
| | 200mg |

**Beispiel H**

Injektionslösungen mit 1mg Wirkstoff pro ml können nach folgender Rezeptur hergestellt werden:

| | |
|---|---|
| Wirkstoff | 1,0mg |
| Polyethylenglykol 400 | 0,3mg |
| Natriumchlorid | 2,7mg |

Wasser zu Injektionszwecken auf 1 ml

Bei der pharmakologischen Prüfung wurden z.B. folgende Ergebnisse erhalten:

1. "Nitrithypoxie"

In diesem Test wird nach der Methode von Gibson und Blass (J. Neurochem.27, 37 (1976)) bei Mäusen mit $NaNO_2$ (250mg/kg s.c.) eine cerebrale Hypoxie erzeugt, die mit dem Tod der Versuchstiere endet. Bestimmt wird, ob die Uberlebenszeit durch Prämedikation mit der Testsubstanz beeinflußt wird. Die erfindungsgemäßen Verbindungen werden dabei in einer Dosis von 100 mg/kg p.o. verabreicht. Die Ergebnisse sind in der folgenden Tabelle angegeben. Bei diesem Test erzielt die bekannte Verbindung Piracetam (= 1-(Aminocarbonylmethyl)-pyrolidin-2-on in einer Dosis von 125 mg/kg p.o. eine prozentuale Verlängerung der Überlebenszeit von 15 %.

**Tabelle**

Prozentuale Verlängerung der Überlebenszeit bei der Verabreichung von 250 mg/kg s.c. $NaNO_2$ und Prämedikation mit Verbindungen der Formel I

| Verbindung gemäß Beispiel | | Prozentuale Verlängerung |
|---|---|---|
| Nr. | 5 | 41 |
| | 13 | 20 |
| | 14 | 20 |
| | 1 | 10 |
| | 11 | 14 |
| | 3 | 23 |
| | 12 | 27 |
| | 2 | 12 |
| | 21d | 29 |
| Piracetam (Vergleich) | | 15 |

2. "Passive avoidance"

Die Testapparatur ist eine Hell-Dunkel-Box mit elektrifizierbarem Gitterboden im dunklen Teil.

90 Minuten nach Verabreichung von Kontroll- und Präparatinjektion werden unerfahrene männliche Mäuse mit Scopolaminhydrobromid (3 mg/kg s.c.) behandelt. 5 Minuten später werden die Mäuse In den hellen Teil der Box gesetzt. Nach dem Überwechseln in den dunklen Teil der Box erhalten sie einen ihnen unangenehmen elektrischen Fußschock. Nach 24 Stunden wird jede Maus einmal in den hellen Teil der Testapparatur gesetzt und die Verweildauer (max. 180 sec.) gemessen. Die mit einer aktiven Dosis eines Präparats und Scopolamin behandelten Tiere zeigen eine lange Verweildauer, ebenso die nicht mit Scopolamin behandelten Tiere, wogegen diejenigen mit Kontrollinjektion und Scopolamin behandelten eine kurze Verweildauer zeigen. Die erfindungsgemäßen Verbingdungen werden dabei in einer Dosis von 3 bis 30 mg/kg p.o. verabreicht. Die Ergebnisse sind in der folgenden Tabelle angegeben. Bei diesem Test erzielt die bekannte Verbindung Piracetam in einer Dosis von 60 mg/kg p.o. eine prozentuale Abschwächung von 100%.

Tabelle

Prozentuale Abschwächung der scopolamininduzierten Amnesie, erkennbar an einer Verlängerung der Zeit bis zum Betreten des dunklen Teils des Passive-Avoidance Testraumes.

| Verbindung gemäß Beispiel Nr. | Dosis (mg/kg) p.o. | Prozentuale Abschwächung |
|---|---|---|
| 5 | 3 | 108 |
| 21d | 30 | 42 |
| 13 | 30 | 36 |
| 14 | 30 | 70 |
| 12 | 30 | 75 |
| 15 | 30 | 111 |
| 19 | 30 | 90 |
| 18 | 3 | 124 |
| 20 | 30 | 108 |
| 2 | 30 | 121 |
| 21 | 30 | 79 |
| Piracetam (Vergleich) | 60 | 100 |

## Ansprüche

1. 2,5-Dimethylpyrrolderivate der Formel I

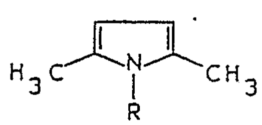

(I)

worin

R Alkyl mit 1 bis 3 C-Atomen, das substituiert ist durch $-NH_2$, Acylamino der Formel

$-NH-CX-R^1$

oder Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, die gegebenenfalls durch eine Carboxylgruppe, Alkoxycarbonyl mit 1 bis 4 C-Atomen in der Alkoxygruppe, Alkyl mit 1 bis 4 C-Atomen oder aliphatische heterocyclische Reste auch durch eine Ketofunktion substituiert sein können; oder Thiophen, Di-oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, die gegebenenfalls durch eine Carboxylgruppe, Alkoxycarbonyl mit 1 bis 4 C-Atomen in der Alkoxygruppe, Alkyl mit 1 bis 4 C-Atomen oder aliphatische heterocyclische Reste auch durch eine Ketofunktion substituiert sein können, bedeutet; X für ein Sauerstoff- oder Schwefelatom steht; $R^1$ Wasserstoff; Alkyl mit 1 bis 5 C-Atomen, das gegebenenfalls substituiert ist durch $-NH_2$, Monoalkylamino mit 1 bis 4 C-Atomen, Dialkylamino mit insgesamt 2 bis 6 C-Atomen, einen N-Pyrrolidinyl-, N-Piperidinyl-, N-Morpholinyl-, N-Thiomorpholinyl- oder durch einen in 4-Stellung gegebenenfalls durch Alkyl mit 1 bis 4 C-Atomen, Phenyl, Toluyl, Chlorphenyl oder Methoxy- oder

Ethoxyphenyl substituierten piperazin-1-yl-Rest, durch Alkoxy mit 1 bis 4 C-Atomen oder durch Phenoxy, das gegebenenfalls substituiert ist durch eine Aminogruppe, Monoalkylamino mit 1 bis 4 C-Atomen, Dialkylamino mit insgesamt 2 bis 6 C-Atomen, Alkanoylamino mit 1 bis 6 C-Atomen, Alkyl mit 1 bis 4 C-Atomen, Alkoxy mit 1 bis 4 C-Atomen, Halogen, Hydroxy, Nitro, Cyan, Carboxy oder Alkoxycarbonyl mit 1 bis 4 C-Atomen in der Alkoxygruppe; Cycloalkyl mit 5 bis 7 C-Atomen;
Phenyl, das gegebenenfalls wie der oben bezeichnete phenoxyrest substituiert sein kann; Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolidin, Oxazol, Oxazolin, Oxazolidin, Thiazol, Thiazolin, Thiazolidin, Pyrimidin, Pyridazin, Pyrazin, Piperazin, Morpholin, Thiomorpholin, Diazepin, Oxazepin, Thiazepin, Perhydrodiazepin, -oxazepin und -thiazepin, die gegebenenfalls durch eine Carboxylruppe, Alkoxycarbonyl mit 1 bis 4 C-Atomen in der Alkoxyruppe, Alkyl mit 1 bis 4 C-Atomen oder aliphatische heterocyclische Reste auch durch eine Ketofunktion substituiert sein können; eine Aminogruppe ($-NH_2$);
oder phenylamino, dessen Phenylkern gegebenenfalls durch Chlor, Alkyl mit 1 oder 2 C-Atomen, Alkoxy mit 1 oder 2 C-Atomen, Carboxy oder Alkoxycarbonyl mit 1 oder 2 C-Atomen in der Alkoxygruppe substituiert sein kann, bedeutet, sowie deren pharmakologisch akzeptablen Säureadditionssalze.

2. 2,5-Dimethylpyrrolderivate gemäß Anspruch 1, dadurch gekennzeichnet, daß R Alkyl mit 1 bis 3 C-Atomen bedeutet, das substituiert ist durch Formylamino, Acetylamino, Propionylamino, Butyrylamino, 4-Chlorphenoxyacetylamino, (2-Oxo-pyrrolidin-1-yl)-acetylamino, N,N-Dimethylaminoacetylamino, L-Thiazolidin-4-yl-carbonylamino, 4-Chlorbenzoylamino, 5-Oxoperhydro-(1,4)-thiazepin-3-yl-carbonylamino, Aminocarbonylamino, 4-Chlorphenylaminocarbonylamino, 1-Acetyl-L-pyrrolidin-2-yl-carbonylamino, 1-Ethyl-(pyrrolidin-2-yl), 2-Oxopyrrolidin1-yl oder 2-Oxo-perhydro-azepin-3-yl.

3. Verfahren zur Herstellung von 2,5-Dimethylpyrrolderivaten der allgemeinen Formel I

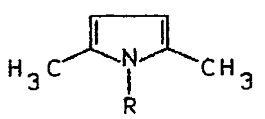

(I),

worin R die in den Ansprüohen 1 oder 2 angegebenen Bedeutungen hat, bzw. ihrer physiologisch verträglichen Säureadditionssalze, dadurch gekennzeichnet, daß man Acetonylaceton mit einem primären Amin der Formel $H_2N-R$ oder einem Säureadditionssalz davon in einem geeigneten Lösungsmittel bei Temperaturen von 20 bis 150 °C, vorzugsweise unterhalb 100 °C, insbesondere bei 40 bis 90 °C, umsetzt und, sofern R ein durch einen Rest der Formel
$-NH-CX-R^1$
substituierter Alkylrest mit 1 bis 3 C-Atomen ist, gewünschtenfalls den Acylrest $-CX-R^1$ in an sich bekannter Weise hydrolytisch abspaltet und/oder gewünschtenfalls die Verbindung in an sich bekannter Weise in ein pharmakologisch verträgliches Säureadditionsalz überführt.

4. Verfahren zur Herstellung von 2,5-Dimethylpyrrolderivaten der Formel I der Ansprüche 1 oder 2, in denen R ein durch eine Acylaminogruppe der Formel $-NH-CO-R^1$ substituierter Alkylrest mit 1 bis 3 C-Atomen ist, dadurch gekennzeichnet, daß man ein Aminoalkylpyrrol der Formel III

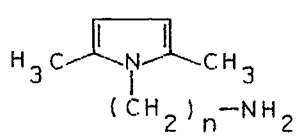

(III),

worin n eine Zahl von 1 bis 3 ist, mit einem reaktiven Carbonsäurederivat, abgeleitet von einer Carbonsäure der Formel $R^1-COOH$, worin $R^1$ die in den Ansprüchen 1 und 2 angegebene Bedeutung hat, oder mit einem Alkalicyanat bzw. -thiocyanat oder mit einem Isocyanat bzw. Isothiocyanat der Formel

$R^3$-NCX,
worin X Sauerstoff oder Schwefel ist und $R^3$ gegebenenfalls substituiertes Phenyl bedeutet, acyliert und gewünschtenfalls die Verbindung in an sich bekannter Weise in ein pharmakologisch verträgliches Säureadditionsalz überführt.

5. Arzneimittelzubereitung enthaltend, neben den üblichen in der Galenik eingesetzten Hilfs- und Trägerstoffen, eine wirksame Dosis eines 2,5-Dimethylpyrrolderivats der Formel I der Ansprüche 1 und/oder 2,

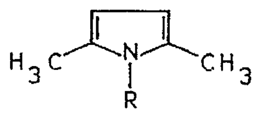

(I),

worin
R die in Anspruch 1 und/oder 2 angegebene Bedeutung hat, oder ein pharmakologisch verträgliches Säureadditionssalz davon und gegebenenfalls ein oder mehrere pharmakologisch wirksame Substanzen in therapeutisch wirksamer Menge.

6. Verwendung der 2,5-Dimethylpyrrolderivate der Formel I der Ansprüche 1 und/oder 2 und ihrer pharmakologisch verträglichen Säureadditionssalze zur Herstellung eines Arzneimittels zur Behandlung von Krankheiten der Gehirnfunktionen wie cerebraler Insuffizienz, cerebraler Alterungsvorgänge und verminderter Gedächtnisleistung.

Patentansprüche
für den Vertragsstaat AT

1. Verfahren zur Herstellung von 2,5-Dimethylpyrrolderivaten der Formel I

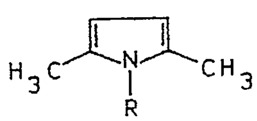

(I)

worin
R Alkyl mit 1 bis 3 C-Atomen, das substituiert ist durch $-NH_2$, Acylamino der Formel
-NH-CX-$R^1$
oder Thiophen, Di- oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, die gegebenenfalls durch eine Carboxylgruppe, Alkoxycarbonyl mit 1 bis 4 C-Atomen in der Alkoxygruppe, Alkyl mit 1 bis 4 C-Atomen oder aliphatische heterocyclische Reste auch durch eine Ketofunktion substituiert sein können; oder Thiophen, Di-oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, die gegebenenfalls durch eine Carboxylgruppe, Alkoxycarbonyl mit 1 bis 4 C-Atomen in der Alkoxygruppe, Alkyl mit 1 bis 4 C-Atomen oder aliphatische heterocyclische Reste auch durch eine Ketofunktion substituiert sein können, bedeutet; X für ein Sauerstoff- oder Schwefelatom steht; $R^1$ Wasserstoff; Alkyl mit 1 bis 5 C-Atomen, das gegebenenfalls substituiert ist durch $-NH_2$, Monoalkylamino mit 1 bis 4 C-Atomen, Dialkylamino mit insgesamt 2 bis 6 C-Atomen, einen N-Pyrrolidinyl-, N-Piperidinyl-, N-Morpholinyl, N-Thiomorpholinyl- oder durch einen in 4-Stellung gegebenenfalls durch Alkyl mit 1 bis 4 C-Atomen, Phenyl, Toluyl, Chlorphenyl oder Methoxy- oder Ethoxyphenyl substituierten Piperazin-1-yl-Rest, durch Alkoxy mit 1 bis 4 C-Atomen oder durch Phenoxy, das gegebenenfalls substituiert ist durch eine Aminogruppe, Monoalkylamino mit 1 bis 4 C-Atomen, Dialkylamino mit insgesamt 2 bis 6 C-Atomen, Alkanoylamino mit 1 bis 6 C-Atomen, Alkyl mit 1 bis 4 C-Atomen, Alkoxy mit 1 bis 4 C-Atomen, Halogen, Hydroxy, Nitro, Cyan, Carboxy oder Alkoxycarbonyl mit 1 bis 4 C-Atomen in der Alkoxygruppe; Cycloalkyl mit 5 bis 7 C-Atomen;
Phenyl, das gegebenenfalls wie der oben bezeichnete Phenoxyrest substituiert sein kann; Thiophen, Di-oder Tetrahydrothiophen, Pyrrol, Pyrrolin, Pyrrolidin, Pyridin, Dihydropyridin, Piperidin, Pyran, Perhydropyran, Oxepin, Thiepin, Azepin, Perhydrooxepin, Perhydrothiepin, Perhydroazepin, Imidazol, Imidazolin, Imidazolidin, Oxazol, Oxazolin, Oxazolidin, Thiazol, Thiazolin, Thiazolidin, Pyrimidin, Pyridazin, Pyrazin, Piperazin, Morpholin, Thiomorpholin, Diazepin, Oxazepin, Thiazepin, Perhydrodiazepin, -oxazepin und -thiazepin, die gegebenenfalls durch eine Carboxylruppe, Alkoxycarbonyl mit 1 bis 4 C-Atomen in der Alkoxyruppe, Alkyl mit 1 bis 4 C-Atomen oder aliphatische heterocyclische Reste auch durch eine ketofunktion substituiert sein können;

eine Aminogruppe ($-NH_2$);

oder Phenylamino, dessen Phenylkern gegebenenfalls durch Chlor, Alkyl mit 1 oder 2 C-Atomen, Alkoxy mit 1 oder 2 C-Atomen, Carboxy oder Alkoxycarbonyl mit 1 oder 2 C-Atomen in der Alkoxygruppe substituiert sein kann, bedeutet, sowie deren pharmakologisch akzeptablen Säureadditionssalze, dadurch gekennzeichnet, daß man Acetonylaceton mit einem primären Amin der Formel $H_2N-R$ oder einem Säureadditionssalz davon in einem geeigneten Lösungsmittel bei Temperaturen von 20 bis 150 ° C umsetzt und, sofern R ein durch einen Rest der Formel

$-NH-CX-R^1$

substituierter Alkylrest mit 1 bis 3 C-Atomen ist, gewünschtenfalls den Acylrest $-CX-R^1$ in an sich bekannter Weise hydrolytisch abspaltet und/oder gewünschtenfalls die Verbindung in an sich bekannter Weise in ein pharmakologisch verträgliches Säureadditionssalz überführt, oder daß man zur Herstellung von Verbindungen der Formel I, in denen R ein durch eine Acylaminogruppe der Formel $-NH-CO-R^1$ substituierter Alkylrest mit 1 bis 3 C-Atomen ist, ein Aminoalkylpyrrol der Formel III

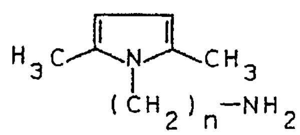

(III),

worin n eine Zahl von 1 bis 3 ist, mit einem reaktiven Carbonsäurederivat, abgeleitet von einer Carbonsäure der Formel $R^1-COOH$, oder mit einem Alkalicyanat bzw. -thiocyanat oder mit einem Isocyanat bzw. Isothiocyanat der Formel

$R^3-NCX$,

worin X Sauerstoff oder Schwefel ist und $R^3$ gegebenenfalls substituiertes Phenyl bedeutet, acyliert.

2. Verfahren nach Anspruch 1, dadurch gekennzeichnet, daß die Ausgangsverbindungen so ausgewählt werden, daß Verbindungen der Formel I entstehen, worin R Alkyl mit 1 bis 3 C-Atomen bedeutet, das substituiert ist durch Formylamino, Acetylamino, Propionylamino, Butyrylamino, 4-Chlorphenoxyacetylamino, (2-Oxo-pyrrolidin-1-yl)-acetylamino, N,N-Dimethylamino-acetylamino, L-Thiazolidin-4-yl-carbonylamino, 4-Chlorbenzoylamino, 5-Oxoperhydro-(1,4)-thiazepin-3-yl-carbonylamino, Aminocarbonylamino, 4-Chlorphenylaminocarbonylamino, 1-Acetyl-L-pyrrolidin-2-yl-carbonylamino, 1-Ethyl(pyrrolidin-2-yl), 2-Oxopyrrolidin1-yl oder 2-Oxo-perhydro-azepin-$^3$-yl.

3. Verfahren nach Anspruch 1 und/oder 2, dadurch gekennzeichnet, daß die Umsetzung des Acetonylacetons mit dem primären Amin der Formel $H_2N-R$ oder einem Säureadditionssalz davon bei Temperaturen unterhalb von 100 ° C durchgeführt wird.

4. Verfahren nach einem oder mehreren der Ansprüche 1 bis 3, dadurch gekennzeichnet, daß die Umsetzung des Acetonylacetons mit dem primären Amin der Formel $H_2N-R$ oder einem Säureadditionssalz davon bei Temperaturen von 40 bis 90 ° C durchgeführt wird.

5. Verfahren zur Herstellung nootrop wirkender Arzneimittelzubereitungen, dadurch gekennzeichnet, daß nach dem in einem oder mehreren der Ansprüche 1 bis 4 angegebenen Verfahren ein 2,5-Dimethylpyrrolderivat der Formel I oder ein pharmakologisch akzeptables Säureadditionssalz davon hergestellt und eine wirksame Dosis davon zusammen mit üblichen, in der Galenik eingesetzten Hilfs- und Trägerstoffen und gegebenenfalls einer oder mehrerer anderer pharmakologisch wirksamer Substanzen in eine Arzneimittelzubereitung überführt wird.

6. Verwendung eines nach einem Verfahren der Ansprüche 1 bis 4 hergestellten 2,5-Dimethylpyrrolderivates der Formel I oder eines pharmakologisch verträglichen Säureadditionssalzes zur Herstellung eines

Arzneimittels zur Behandlung von Krankheiten der Gehirnfunktionen wie cerebraler Insuffizienz, cerebraler Alterungsvorgänge und verminderter Gedächtsnisleistung.

## Claims

**1.** 2,5-Dimethylpyrrole derivatives of the formula I

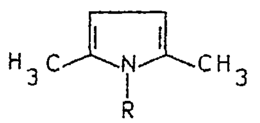

(I)

in which R denotes alkyl having 1 to 3 C atoms which is substituted by $-NH_2$, acylamino of the formula $-NH-CX-R^1$

or thiophene, di- or tetrahydrothiophene, pyrrole, pyrroline, pyrrolidine, pyridine, dihydropyridine, piperidine, pyran, perhydropyran, oxepine, thiepine, azepine, perhydrooxepine, perhydrothiepine, perhydroazepine, which may optionally be substituted by a carboxyl group, alkoxycarbonyl having 1 to 4 C atoms in the alkoxy group, alkyl having 1 to 4 C atoms, or in the case of aliphatic heterocyclic radicals may be substituted by a ceto function; or thiophene, di-or tetrahydrothiophene, pyrrole, pyrroline, pyrrolidine, pyridine, dihydropyridine, piperidine, pyran, perhydropyran, oxepine, thiepine, azepine, perhydrooxepine, perhydrothiepine, perhydroazepine, which may be substituted by a carboxyl group, alkoxycarbonyl having 1 to 4 C atoms in the alkoxy group, alkyl having 1 to 4 C atoms or in the case of aliphatic heterocyclic radicals may be substituted by a ceto function;

X denotes an oxygen or sulphur atom;

$R^1$ denotes hydrogen; alkyl which has 1 to 5 C atoms and is optionally substituted by $-NH_2$, monoalkylamino having 1 to 4 C atoms, dialkylamino having a total of 2 to 6 C atoms, an N-pyrrolidinyl, N-piperidinyl, N-morpholinyl or N-thiomorpholinyl radical, or by a 1-piperazinyl radical which is substituted in the 4-position optionally by alkyl having 1 to 4 C atoms, phenyl, toluyl, chlorophenyl or methoxyphenyl or ethoxyphenyl, or by alkoxy having 1 to 4 C atoms or by phenoxy which is optionally substituted by an amino group, monoalkylamino having 1 to 4 C atoms, dialkylamino having a total of 2 to 6 C atoms, alkanoylamino having 1 to 6 C atoms, alkyl having 1 to 4 C atoms, alkoxy having 1 to 4 C atoms, halogen, hydroxy, nitro, cyano, carboxy or alkoxycarbonyl having 1 to 4 C atoms in the alkoxy group; cycloalkyl having 5 to 7 C atoms; phenyl which may optionally be substituted like the phenoxy radical mentioned above; thiophene, di-or tetrahydrothiophene, pyrrole, pyrroline, pyrrolidine, pyridine, dihydropyridine, piperidine, pyran, perhydropyran, oxepine, thiepine, azepine, perhydrooxepine, perhydrothiepine, perhydroazepine, imidazole, imidazoline, imidazolidine, oxazole, oxazoline, oxazolidine, thiazole, thiazoline, thiazolidine, pyrimidine, pyridazine, pyrazine, piperazine, morpholine, thiomorpholine, diazepine, oxapezine, thiazepine, perhydrodiazepine, -oxazepine and -thiazepine, which may optionally be substituted by a carboxyl group, alkoxycarbonyl having 1 to 4 C atoms in the alkoxy group, alkyl having 1 to 4 C atoms or in the case of aliphatic heterocyclic radicals may be substituted by a ceto function; an amino group ($-NH_2$); or phenylamino, the phenyl nucleus of which may optionally be substituted by chlorine, alkyl having 1 or 2 C atoms, alkoxy having 1 or 2 C atoms, carboxy or alkoxycarbonyl having 1 or 2 C atoms in the alkoxy group, as well as their pharmacologically acceptable acid addition salts.

**2.** 2,5-Dimethylpyrrole derivatives according to claim 1, characterized in that R denotes alkyl which has 1 to 3 C atoms and is substituted by formylamino, acetylamino, propionylamino, butyrylamino, 4-chlorophenoxyacetylamino, (2-oxo-1-pyrrolidinyl)acetylamino, N,N-dimethylaminoacetylamino, L-thiazolidin-4-yl-carbonylamino, 4-chlorobenzoylamino, 5-oxoperhydro-(1,4)-thiazepin-3-yl-carbonylamino, aminocarbonylamino, 4-chlorophenylaminocarbonylamino, 1-acetyl-L-pyrrolidin-2-yl-carbonylamino, 1-ethyl-2-pyrrolidinyl, 2-oxo-1-pyrrolidinyl or 2-oxoperhydro-3-azepinyl.

**3.** Process for the preparation of 2,5-dimethylpyrrole derivatives of the general formula I

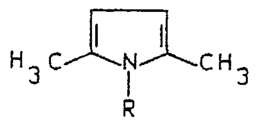

(I)

in which R has the meanings indicated in claim 1 or 2, and of their physiologically tolerated acid addition salts, characterized in that acetonylacetone is reacted with a primary amine of the formula $H_2N$-R, or an acid addition salt thereof, in a suitable solvent at temperatures from 20 to 150 °C, preferably below 100 °C, in particular at 40 to 90 °C, and, where R is an alkyl radical which has 1 to 3 C atoms and is substituted by a radical of the formula

-NH-CX-$R^1$,

if desired, the acyl radical -CX-$R^1$ is eliminated by hydrolysis in a manner known per se, and/or, if desired, the compound is converted in a manner known per se into a pharmacologically tolerated acid addition salt.

**4.** Process for the preparation of 2,5-dimethylpyrrole derivatives of the formula I of claim 1 or 2, in which R is an alkyl radical which has 1 to 3 C atoms and is substituted by an acylamino group of the formula -NH-CO-$R^1$, characterized in that an aminoalkylpyrrole of the formula II

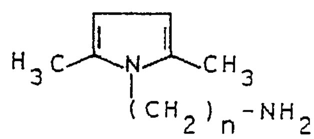

(II)

in which n is a number from 1 to 3, is acylated with a reactive carboxylic acid derivative derived from a carboxylic acid cf the formula $R^1$-COOH, in which $R^1$ has the meaning indicated in claims 1 and 2, or with an alkali metal cyanate or thiocyanate or with an isocyanate or isothiocyanate of the formula

$R^3$-NCX

in which X is oxygen or sulphur, and $R^3$ denotes optionally substituted phenyl; and, if desired, the compound is converted in a manner known per se, into a pharmacologically tolerated acid addition salt.

**5.** Medicament composition containing, in addition to the auxiliaries and vehicles customarily used in pharmacy, an active dose of a 2,5-dimethylpyrrole derivative of the formula I of Claim 1 and/or 2,

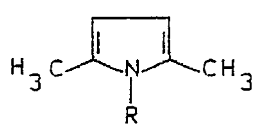

(I)

in which R has the meaning indicated in claim 1 and/or 2, or a pharmacologically tolerated acid addition salt thereof and optionally one or several pharmacologically active substances in therapeutically active amounts.

**6.** Use of the 2,5-dimethylpyrrole derivatives of the formula I of claim 1 or 2 and of their pharmacologically tolerated acid addition salts for treating diseases of the brain functions such as cerebral insufficiency, cerebral aging processes and reduced memory performance.

Claims for the Contracting State AT

**1.** Process for preparing 2,5-dimethylpyrrole derivatives of the formula I

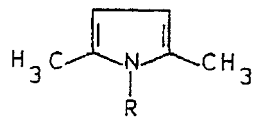

( I )

in which R denotes alkyl having 1 to 3 C atoms which is substituted by $-NH_2$, acylamino of the formula -NH-CX-R[1] or thiophene, di- or tetrahydrothiophene, pyrrole, pyrroline, pyrrolidine, pyridine, dihydropyridine, piperidine, pyran, perhydropyran, oxepine, thiepine, azepine, perhydrooxepine, perhydrothiepine, perhydroazepine, which may optionally be substituted by a carboxyl group, alkoxycarbonyl having 1 to 4 C atoms in the alkoxy group, alkyl having 1 to 4 C atoms, or in the case of aliphatic heterocyclic radicals may be substituted by a ceto function; or thiophene, di-or tetrahydrothiophene, pyrrole, pyrroline, pyrrolidine, pyridine, dihydropyridine, piperidine, pyran, perhydropyran, oxepine, thiepine, azepine, perhydrooxepine, perhydrothiepine, perhydroazepine, which may optionally be substituted by a carboxyl group, alkoxycarbonyl having 1 to 4 C atoms in the alkoxy group, alkyl having 1 to 4 C atoms or in the case of aliphatic heterocyclic radicals may be substituted by a ceto function;
X denotes an oxygen or sulphur atom;
$R^1$ denotes hydrogen; alkyl which has 1 to 5 C atoms and is optionally substituted by $-NH_2$, monoalkylamino having 1 to 4 C atoms, dialkylamino having a total of 2 to 6 C atoms, an N-pyrrolidinyl, N-piperidinyl, N-morpholinyl or N-thiomorpholinyl radical, or by a 1-piperazinyl radical which is substituted in the 4-position optionally by alkyl having 1 to 4 C atoms, phenyl, toluyl, chlorophenyl or methoxyphenyl or ethoxyphenyl, or by alkoxy having 1 to 4 C atoms or by phenoxy which is optionally substituted by an amino group, monoalkylamino having 1 to 4 C atoms, dialkylamino having a total of 2 to 6 C atoms, alkanoylamino having 1 to 6 C atoms, alkyl having 1 to 4 C atoms, alkoxy having 1 to 4 C atoms, halogen, hydroxy, nitro, cyano, carboxy or alkoxycarbonyl having 1 to 4 C atoms in the alkoxy group; cycloalkyl having 5 to 7 C atoms; phenyl which may optionally be substituted like the phenoxy radical mentioned above; thiophene, di-or tetrahydrothiophene, pyrrole, pyrroline, pyrrolidine, pyridine, dihydropyridine, piperidine, pyran, perhydropyran, oxepine, thiepine, azepine, perhydrooxepine, perhydrothiepine, perhydroazepine, imidazole, imidazoline, imidazolidine, oxazole, oxazoline, oxazolidine, thiazole, thiazoline, thiazolidine, pyrimidine, pyridazine, pyrazine, piperazine, morpholine, thiomorpholine, diazepine, oxapezine, thiazepine, perhydrodiazepine, -oxazepine and -thiazepine, which may optionally be substituted by a carboxyl group, alkoxycarbonyl having 1 to 4 C atoms in the alkoxy group, alkyl having 1 to 4 C atoms or in the case of aliphatic heterocyclic radicals may be substituted by a ceto function; an amino group ($-NH_2$); or phenylamino, the phenyl nucleus of which may optionally be substituted by chlorine, alkyl having 1 or 2 C atoms, alkoxy having 1 or 2 C atoms, carboxy or alkoxycarbonyl having 1 or 2 C atoms in the alkoxy group, as well as their pharmacologically acceptable acid addition salts, characterized in that acetonylacetone is reacted with a primary amine of the formula $H_2N-R$, or an acid addition salt thereof, in a suitable solvent at temperatures from 20 to 150 °C and, where R is an alkyl radical which has 1 to 3 C atoms and is substituted by a radical of the formula

$-NH-CX-R^1$,

if desired, the acyl radical $-CX-R^1$ is eliminated by hydrolysis in a manner known per se, and/or, if desired, the compound is converted in a manner known per se into a pharmacologically tolerated acid addition salt, or that for preparing compounds of the formula I, in which R is an alkyl radical which has 1 to 3 C atoms and is substituted by an acylamino group, an aminoalkylpyrrole of the formula III

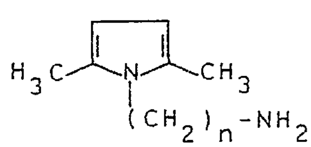

( III )

in which n is a number from 1 to 3, is acylated with a reactive carboxylic acid derivative derived from a carboxylic acid of the formula $R^1$-COOH, or with an alkali metal cyanate or thiocyanate or with an isocyanate or isothiocyanate of the formula

$R^3$-NCX

in which X is oxygen or sulphur, and $R^3$ denotes optionally substituted phenyl.

**2.** Process of claim 1, characterized in that the starting compounds are selected such that compounds of the formula I are formed, wherein R denotes alkyl which has 1 to 3 C atoms and is substituted by fomylamino, acetylamino, propionylamino, butyrylamino, 4-chlorophenoxyacetylamino, (2-oxo-1-pyrrolidinyl)acetylamino, N,N-dimethylaminoacetylamino, L-thiazolidin-4-yl-carbonylamino, 4-chlorobenzoylamino, 5-oxoperhydro-(1,4)-thiazepin-3-yl carbonylamino, aminocarbonylamino, 4-chlorophenylaminocarbonylamino, 1-acetyl-L-pyrrolidin-2-yl-carbonylamino, 1-ethyl-2-pyrrolidinyl, 2-oxo-1-pyrrolidinyl or 2-oxoperhydro-3-azepinyl.

**3.** Process of claim 1 and/or 2, characterized in that the reaction of acetonylacetone with the primary amine of the formula $H_2N$-R or an acid addition salt thereof is effected at temperatures below 100 °C.

**4.** Process of one or more of olaims 1 to 3, characterized in that the reaction of acetonylacetone with the primary amine of the formula $H_2N$-R or an acid addition salt thereof is effected at temperatures of 40 to 90 °C.

**5.** Process for preparing nootropically acting medicament compositions, characterized in that according to a method indicated in one or more of claims 1 to 4 a 2,5-dimethylpyrrole derivative or a pharmacologically acceptable acid addition salt thereof is prepared and an effective dose thereof is converted together with auxiliaries and carriers customarily used in pharmacy and optionally one or more other pharmacologically acting substances, into a medicament composition.

**6.** Use of a 2,5-dimethylpyrrole derivative of the formula I prepared by a method of claims 1 to 4 and of a pharmacologically tolerated acid addition salt for treating diseases of the brain functions such as cerebral insufficiency, cerebral aging processes and reduced memory performance.

## Revendications

**1.** Dérivés du diméthyl-2,5 pyrrole répondant à la formule I:

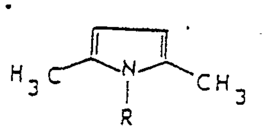

(I)

dans laquelle:

R représente:

- un alkyle qui contient de 1 à 3 atomes de carbone et qui porte un radical $-NH_2$, un radical acylamino de formule :

  $-NH-CX-R^1$

  ou un radical de thiophene, de di- ou tétrahydrothiophène, de pyrrole, de Pyrroline, de pyrrolidine, de pyridine, de dihydropyridine, de pipéridine, de pyranne, de perhydropyranne, d'oxépinne, de thiépinne, d'azépine, de perhydro-oxépinne, de perhydrothiépinne ou de perhydro-azépine, chacun de ces radicaux étant éventuellement porteur d'un carboxy, d'un alcoxycarbonyle contenant de 1 à 4 atomes de carbone dans sa partie alcoxy, d'un alkyle contenant de 1 à 4 atomes de carbone ou, dans le cas des radicaux hétérocycliques aliphatiques, également d'une fonction cétonique; ou
- un radical de thiophène, de di- ou tétrahydrothiophène, de pyrrole, de pyrroline, de pyrrolidine, de pyridine, de dihydropyridine, de pipéridine, de pyranne, de perhydropyranne, d'oxépinne, de thiépinne, d'azépine, de perhydro-oxépinne, de perhydrothiépinne ou de perhydro-azépine, chacun de ces radicaux étant éventuellement porteur d'un carboxy, d'un alcoxycarbonyle contenant de 1 à 4 atomes de carbone ou, dans le cas des radicaux hétérocycliques aliphatiques, également d'une fonction cétonique, X représente un atome d'oxygène ou de soufre, $R^1$ représente :

- l'hydrogène ;
- un alkyle qui contient de 1 à 5 atomes de carbone et qui porte éventuellement un $-NH_2$, un mono-alkylamino contenant de 1 à 4 atomes de carbone, un dialkylamino contenant au total de 2 à 6 atomes de carbone, un N-pyrrolidinyle, un N-pipéridinyle, un N-morpholinyle, un N-thiomorpholinyle, un radical pipérazinyle-1 éventuellement porteur, en sa position 4, d'un alkyle en $C_1$-$C_4$, d'un phényle, d'un toluyle, d'un chlorophényle, d'un méthoxy ou d'un éthoxy, un alcoxy contenant de 1 à 4 atomes de carbone ou un phénoxy éventuellement porteur d'un amino, d'un mono-alkylamino contenant de 1 à 4 atomes de carbone, d'un dialkylamino contenant au total de 2 à 6 atomes de carbone, d'un alcanoylamino contenant de 1 à 6 atomes de carbone, d'un alkyle contenant de 1 à 4 atomes de carbone, d'un alcoxy contenant de 1 à 4 atomes de carbone, d'un halogène, d'un hydroxy, d'un nitro, d'un cyano, d'un carboxy ou d'un alcoxycarbonyle contenant de 1 à 4 atomes de carbone dans sa partie alcoxy;
- un cycloalkyle contenant de 5 à 7 atomes de carbone;
- un phényle qui peut éventuellement être substitué comme le radical phénoxy défini ci-dessus;
- un radical de thiophène, de di- ou tétrahydrothiophène, de pyrrole, de pyrroline, de pyrrolidine, de pyridine, de dihydropyridine, de pipéridine, de pyranne, de perhydropyranne, d'oxépinne, de thiépinne, d'azépine, de perhydro-oxépinne, de perhydrothiépinne, de perhydro-azépine, d'imidazole, d'imidazoline, d'imidazolidine, d'oxazole, d'oxazoline, d'oxazolidine, de thiazole, de thiazoline, de thiazolidine, de pyrimidine, de pyridazine, de pyrazine, de pipérazine, de morpholine, de thiomorpholine, de diazépine, d'oxazépine, de thiazépine, de perhydro-diazépine, de perhydro-oxazépine ou de perhydro-thiazépine, chacun de ces radicaux pouvant porter un carboxy, un alcoxycarbonyle renfermant de 1 à 4 atomes de carbone dans sa partie alcoxy, un alkyle contenant de 1 à 4 atomes de carbone ou, dans le cas radicaux hétérocycliques aliphatiques, également une fonction cétonique ;
- un radical amino $-NH_2$; ou
- un radical phénylamino dont le noyau phényle porte éventuellement du chlore, un alkyle à 1 ou 2 atomes de carbone, un alcoxy à 1 ou 2 atomes de carbone, un carboxy ou un alcoxycarbonyle dont la partie alcoxy contient 1 ou 2 atomes de carbone, ainsi que les sels d'addition acceptables du point de vue pharmacologique que ces composés forment avec des acides.

**2.** Dérivés du diméthyl-2,5 pyrrole selon la revendication 1, caractérisés en ce que R représente un radical alkyle qui contient de 1 à 3 atomes de carbone et qui porte, comme substituant, un radical formylamino, acétylamino, propionylamino, butyrylamino, (chloro-4 phényl)-acétylamino, (oxo-2 pyrrolidinyl-1)-acétylamino, N,N-diméthylaminoacétylamino, (L-thiazolidinyl-4)-carbonylamino, chloro-4 benzoylamino, (oxo-5 perhydrothiazépine-l,4 yl)-3 carbonylamino, aminocarbonylamino, (chloro-4 phényl)-aminocarbonylamino, (acétyl-1 L-pyrrolidinyl-2)-carbonylamino, éthyl-1 pyrrolidinyle-2, oxo-2 pyrrolidinyle-1 ou oxo-2 perhydro-azépinyle-3.

**3.** Procédé pour préparer des dérivés du diméthyl-2,5 pyrrole répondant à la formule I:

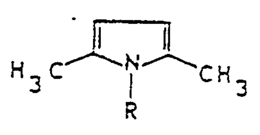

(I),

dans laquelle R a les significations qui lui ont été données dans les revendications 1 et 2, ou les sels d'addition qu'ils forment avec des acides acceptables du point de vue physiologique, procédé caractérisé en ce qu'on fait réagir l'acétonyl-acétone avec une amine primaire de formule $H_2N$-R, ou avec un sel d'addition d'acide d'une telle amine, dans un solvant approprié, à des températures de 20 à 150°C, de préférence à des températures inférieures à 100°C, plus particulièrement comprises entre 40 et 90°C, et, lorsque R représente un radical alkyle contenant de 1 à 3 atomes de carbone et porteur d'un radical de formule:

$-NH-CX-R^1$,

on élimine, si on le désire, le radical acyle $-CX-R^1$ par hydrolyse , de manière connue, et/ou, si on le désire, on transforme le composé, de manière connue, en un sel d'addition d'acide acceptable du point de vue pharmacologique.

4. Procédé pour préparer des dérivés du diméthyl-2,5 pyrrole de formule I selon l'une des revendications 1 et 2 dans lesquels R représente un radical alkyle qui contient de 1 à 3 atomes de carbone et qui porte un radical acylamino de formule -NH-CO-R[1], procédé caractérisé en ce qu'on acyle un aminoalkyl-pyrrole répondant à la formule III:

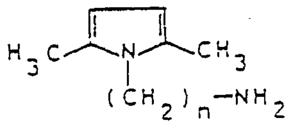

(III),

dans laquelle n désigne un nombre de 1 à 3, avec un dérivé réactif d'acide carboxylique provenant d'un acide carboxylique de formule $R^1$-COOH dans lequel $R^1$ a la signification donnée dans les revendications 1 et 2, ou avec un cyanate ou un thiocyanate de métal alcalin ou avec un isocyanate ou un isothiocyanate répondant à la formule :

$R^3$-NCX

dans laquelle X représente l'oxygène ou le soufre et $R^3$ un phényle éventuellement substitué, et, si on le désire, on transforme le composé, de manière connue, en un sel d'addition d'acide acceptable du point de vue pharmacologique.

5. Médicament contenant, en plus d'excipients et adjuvants pris parmi ceux dont on se sert ordinairement en pharmacie, une dose efficace d'un dérivé du diméthyl-2,5 pyrrole de formule I selon l'une des revendications 1 et 2, c'est-à-dire d'un composé répondant à la formule I:

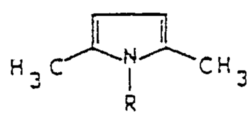

(I),

dans laquelle R a la signification qui lui a été donnée dans les revendications 1 et 2, ou d'un de ses sels d'addition d'acides acceptables du point de vue pharmacologique, et éventuellement une ou plusieurs autres substances douées d'une activité pharmacologique, à une dose suffisante pour avoir une action thérapeutique.

6. Application des dérivés du diméthyl-2,5 pyrrole de formule I selon l'une des revendications 1 et 2, et de leurs sels d'addition d'acides acceptables du point de vue pharmacologique, à la fabrication d'un médicament pour le traitement de maladies affectant les fonctions cérébrales, telles que l'insuffisance cérébrale, les phénomènes de sénescence cérébrale et l'affaiblissement de la mémoire.

REVENDICATIONS (pour l'Etat contractant AT)

1. Procédé pour préparer des dérivés du diméthyl-2,5 pyrrole répondant à la formule I:

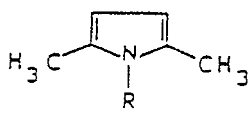

(I)

dans laquelle :

R représente:

- un alkyle qui contient de 1 à 3 atomes de carbone et qui porte un radical $-NH_2$, un radical acylamino de formule :

  $-NH-CX-R^1$

  ou un radical de thiophène, de di- ou tétrahydrothiophène, de pyrrole, de pyrroline, de pyrrolidine, de pyridine, de dihydropyridine, de pipéridine, de pyranne, de perhydropyranne, d'oxépinne, de

thiépinne, d'azépine, de perhydro-oxépinne, de perhydrothiépinne ou de perhydro-azépine, chacun de ces radicaux étant éventuellement porteur d'un carboxy, d'un alcoxycarbonyle contenant de 1 à 4 atomes de carbone dans sa partie alcoxy, d'un alkyle contenant de 1 à 4 atomes de carbone ou, dans le cas des radicaux hétérocycliques aliphatiques également d'une fonction cétonique; ou

- un radical de thiophène, de di- ou tétrahydrothiophène, de pyrrole, de pyrroline, de pyrrolidine, de pyridine, de dihydropyridine, de pipéridine, de pyranne, de perhydropyranne, d'oxépinne, de thiépinne, d'azépine, de perhydro-oxépinne, de perhydrothiépinne ou de perhydro-azépine, chacun de ces radicaux étant éventuellement porteur d'un carboxy, d'un alcoxycarbonyle contenant de 1 à 4 atomes de carbone, dans sa partie alcoxy, d'un alkyle contenant de 1 à 4 atomes de carbone ou, dans le cas des radicaux hétérocycliques aliphatique, également d'une fonction cétonique, X représente un atome d'oxygène ou de soufre, $R^1$ représente :
- l'hydrogène ;
- un alkyle qui contient de 1 à 5 atomes de carbone et qui porte éventuellement un $-NH_2$, un mono-alkylamino contenant de 1 à 4 atomes de carbone, un dialkylamino contenant au total de 2 à 6 atomes de carbone, un N-pyrrolidinyle, un N-pipéridinyle, un N-morpholinyle, un N-thiomorpholinyle, un radical pipérazinyle-l éventuellement porteur, en sa position 4, d'un alkyle en $C_1$-$C_4$, d'un phényle, d'un toluyle, d'un chlorophényle, d'un méthoxy ou d'un éthoxy, un alcoxy contenant de 1 à 4 atomes de carbone ou un phénoxy éventuellement porteur d'un amino, d'un mono-alkylamino contenant de 1 à 4 atomes de carbone, d'un dialkylamino contenant au total de 2 à 6 atomes de carbone, d'un alcanoylamino contenant de 1 à 6 atomes de carbone, d'un alkyle contenant de 1 à 4 atomes de carbone, d'un alcoxy contenant de 1 à 4 atomes de carbone, d'un halogène, d'un hydroxy, d'un nitro, d'un cyano, d'un carboxy ou d'un alcoxycarbonyle contenant de 1 à 4 atomes de carbone dans sa partie alcoxy ;
- un cycloalkyle contenant de 5 à 7 atomes de carbone;
- un phényle qui peut éventuellement être substitué comme le radical phénoxy défini ci-dessus ;
- un radical de thiophène, de di- ou tétrahydrothiophène, de pyrrole, de pyrroline, de pyrrolidine, de pyridine, de dihydropyridine, de pipéridine, de pyranne, de perhydropyranne, d'oxépinne, de thiépinne, d'azépine, de perhydro-oxépinne, de perhydrothiépinne, de perhydro-azépine, d'imidazole, d'imidazoline, d'imidazolidine, d'oxazole, d'oxazoline, d'oxazolidine, de thiazole, de thiazoline, de thiazolidine, de pyrimidine, de pyridazine, de pyrazine, de pipérazine, de morpholine, de thiomorpholine, de diazépine, d'oxazépine, de thiazépine, de perhydro-diazépine, de perhydro-oxazépine ou de perhydro-thiazépine, chacun de ces radicaux pouvant porter un carboxy, un alcoxycarbonyle renfermant de 1 à 4 atomes de carbone dans sa partie alcoxy, un alkyle contenant de 1 à 4 atomes de carbone ou, dans le cas des radicaux hétérocycliques aliphatiques, également une fonction cétonique;
- un radical amino $-NH_2$; ou
- un radical phénylamino dont le noyau phényle porte éventuellement du chlore, un alkyle à 1 ou 2 atomes de carbone, un alcoxy à 1 ou 2 atomes de carbone, un carboxy ou un alcoxycarbonyle dont la partie alcoxy contient 1 ou 2 atomes de carbone, ainsi que les sels d'addition acceptables du point de vue pharmacologique que ces composés forment avec des acides, procédé caractérisé en ce qu'on fait réagir l'acétonyl-acétone avec une amine primaire de formule $H_2N-R$, ou avec un sel d'addition d'acide d'une telle amine, dans un solvant approprié, à des températures de 20 à 150 °C, et, lorsque R représente un radical alkyle contenant de 1 à 3 atomes de carbone et portant un radical de formule :

$-NH-CX-R^1$,

on élimine, si on le désire, le radical acyle $-CX-R^1$ par hydrolyse, de manière connue, et/ou, si on le désire, on transforme le composé, de manière connue, en un sel d'addition d'acide acceptable du point de vue pharmacologique, ou, pour préparer des composés de formule I dans lesquels R représente un radical alkyle qui contient de 1 à 3 atomes de carbone et qui porte un radical acylamino de formule $-NH-CO-R^1$, on acyle un aminoalkyl-pyrrole répondant à la formule III :

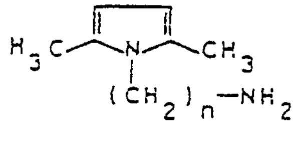

(III),

dans laquelle n désigne un nombre entier de 1 à 3, avec un dérivé réactif d'acide carboxylique provenant d'un acide carboxylique de formule $R^1$-COOH, ou avec un cyanate ou un thiocyanate de métal alcalin, ou avec un isocyanate ou un isothiocyanate répondant à la formule :

$R^3$-NCX

dans laquelle X représente l'oxygène ou le soufre et $R^3$ représente un radical phényle éventuellement substitué.

2. Procédé selon la revendication 1 caractérisé en ce qu'on choisit les corps de départ de telle façon qu'il se forme des composés de formule I dans lesquels R représente un alkyle contenant de 1 à 3 atomes de carbone qui porte, comme substituant, un radical formylamino, acétylamino, propionylamino, butyrylamino, (chloro-4 phénoxy)-acétylamino, (oxo-2 pyrrolidinyl-1)-acétylamino, N,N-diméthylaminoacétylamino, (L-thiazolidinyl-4)-carbonyl-amino, chloro-4 benzoylamino, (oxo-5 perhydrothiazépine-1,4 yl-3)-carbonylamino, aminocarbonylamino, (chloro-4 phényl)-aminocarbonylamino, (acétyl-1 L-pyrrolidinyl-2)-carbonylamino, éthyl-1 pyrrolidinyle-2, oxo-2 pyrrolidinyle-1 ou oxo-2 perhydro-azépinyle-3.

3. Procédé selon l'une des revendications 1 et 2, caractérisé en ce que la réaction de l'acétonylacétone avec l'amine primaire de formule $H_2N$-R ou avec un sel d'addition d'acide d'une telle amine est effectuée à des températures inférieures à 100 °C.

4. Procédé selon une ou plusieurs des revendications 1 à 3, caractérisé en ce que la réaction de l'acétonylacétone avec l'amine primaire de formule $H_2N$-R ou avec un sel d'addition d'acide d'une telle amine est effectuée à des températures de 40 à 90 °C.

5. Procédé pour préparer des médicaments à action nootrope, procédé caractérisé en ce qu'on prépare, par le procédé indiqué dans une ou plusieurs des revendications 1 à 4, un dérivé du diméthyl-2,5 pyrrole de formule I, ou un sel, pharmacologiquement acceptable, formé par addition d'un acide sur un tel dérivé du pyrrole, et on fabrique un médicament avec une dose efficace d'un composé de ce genre, associé à des adjuvants et excipients usuels utilisés en pharmacie et, éventuellement, à une ou plusieurs autres substances douées d'une activité pharmacologique.

6. Application d'un dérivé du diméthyl-2,5 pyrrole, ou d'un de ses sels d'addition d'acides acceptables du point de vue pharmacologique, qui ont été préparés par un procédé selon l'une des revendications 1 à 4, pour la fabrication d'un médicament destiné au traitement de maladies affectant les fonctions cérébrales, telles que l'insuffisance cérébrale, des phénomènes de sénescence cérébrale et l'affaiblissement de la mémoire.